# EUROPEAN PATENT APPLICATION

(11) **EP 2 450 051 A1**
(43) Date of publication of application: **09.05.2012**
(21) Application number: 10794066.0
(22) Date of filing: 24.06.2010
(51) Int. Cl.: A61K 38/21, A61K 31/56, A61K 31/7056, A61P 1/16, A61P 31/14, A61P 43/00

(54) **MEDICINAL AGENT AND METHOD FOR TREATMENT OF INTRACTABLE CHRONIC HEPATITIS C**

(30) Priority: 30.06.2009 JP 2009155405
(71) Applicant: Meiji Seika Pharma Co., Ltd., Tokyo 104-8002 (JP)
(72) Inventor: NOMOTO Kozo, Tokyo 104-8002 (JP); MATSUI Daisuke, Tokyo 104-8002 (JP); HIRANUMA Toyokazu, Tokyo 104-8002 (JP)
(74) Representative: Cohausz & Florack
(86) International application number: PCT/JP2010/060762
(87) International publication number: WO 2011/001897

(57) **Abstract**

It is found that even refractory chronic hepatitis C can be treated effectively and safely by administering an effective amount of a combination of 22β-methoxyolean-12-ene-3β,24(4β)-diol with an IFN.

## Description

### [Technical Field]

The present invention relates to an agent for treating refractory chronic hepatitis C, the agent comprising a combination of 22β-methoxyolean-12-ene-3β,24(4β)-diol with an interferon as active ingredients, the combination administered in an effective amount. The present invention also relates to a method for treating refractory chronic hepatitis C using the agent.

### [Background Art]

In Japan, an estimated 1.5 to 2.0 million people are persistently infected with hepatitis C virus (HCV), and it is assumed that most of them have chronic hepatitis C showing the symptoms of chronic hepatitis. Based on observations of liver tissue, 80 to 90% of HCV-infectedpeople have chronic hepatitis C, and there is a possibility that 50 to 60% of these infected people develop cirrhosis or hepatocellular carcinoma via chronic hepatitis. Liver cancer is caused by HCV infection in many cases, and the number of deaths by liver cancer is approximately 30,000 per year. Meanwhile, in other countries, although differing from country to country, an estimated 4.1 million people are infected with HCV (3.2 million people have chronic hepatitis) in the United States, and approximately 9.0 million people in Europe. Hepatitis C has become a major clinical problem worldwide. A patient who is a subject of a chronic hepatitis C treatment is a patient infected with HCV. There are 6 major types of HCV genotype from genotype 1 to genotype 6. It is known that particularly genotype 1 and genotype 2 are widespread worldwide (Non Patent Literature 1). Furthermore, these genotypes include subtypes, which can be distinguished from each other according to the base sequence at a specific region of RNAs constituting the HCV, and the HCV load can also be measured (Non Patent Literature 2).

In treatment against chronic hepatitis C, interferons (IFNs) are widely used to eliminate HCV from the body. IFNs are bioactive substances produced by an innate immune system at the time of virus infection, and have an antiviral activity to inhibit viral replication. IFNs are species-specific proteins with a high degree of homology to each other. It is known that there are four classes of IFNs in humans. At present, as pharmaceutical products, several different types of IFN-α and IFN-β including PEGylated IFN (PEG-IFN) type are approved as drugs exhibiting effects on chronic hepatitis C. PEG-IFN is an IFN with polyethylene glycol (PEG) attached thereto for extending the time of being sustained in a body.

It is known that the HCV load and the HCV genotype in the blood of a patient are important factors affecting the effects of IFNs on chronic hepatitis C. It has been revealed that the effects are the lowest when the HCV load in the blood of a patient is a high viral load (1 Meq/mL, 5.0 Log IU/mL (100 KIU/mL) or 300 fmol/L or higher) or when the HCV genotype is genotype 1, especially genotype 1b (Non Patent Literature 3).

At present, a combination therapy of PEG-IFN with ribavirin for 48 weeks is a standard therapy for a patient with genotype 1b chronic hepatitis C and having a high viral load. The highest effect is expected from the combination therapy. However, the effect is only approximately around 50% for a patient with genotype 1b and having a high viral load who is difficult to treat the most (Non Patent Literature 3). This is considered to be, besides the virus-side factor of genotype 1b exhibiting treatment resistance to the standard therapy, mainly from an influence of ribavirin that causes side effects such as anemia. For this reason, the drug dose needs to be reduced. Thus, the therapeutic effect becomes insufficient, so that the treatment is discontinued or terminated (Non Patent Literatures 1, 4). The frequencies of the side effects observed in the standard therapy are as high as 56.2 to 84.9% for hemoglobin reduction, and 52.6 to 82.9% for erythrocyte reduction. Anemia is listed in the top of the important side effects of ribavirin (Non Patent Literatures 5, 6). In fact, the frequency of reducing the ribavirin amount or discontinuation is as high as 31.1% for those who are under 60 years old and 69.4% for those who are 60 years old or above (Non Patent Literature 4). For this reason, when ribavirin is used in the treatment, whether the blood hemoglobin value is 12 g/dL or above has to be checked before the treatment is started. If a patient does not have a heart disease now or in the past, when the blood hemoglobin value is below 10 g/dL, the amount of ribavirin administered is reduced, or when the value is below 8.5 5 g/dL, the administration is terminated. Meanwhile, if a patient has a heart disease now or in the past, in addition to the above baseline, when the hemoglobin value drops down 2 g/dL or more from the value before the treatment is started, or when the hemoglobin value is below 12 g/dL, the amount of ribavirin administered is reduced or the administration is terminated (Non Patent Literatures 5, 6). In this manner, when the amount of ribavirin administered is reduced or the administration is terminated to reduce the side effects of ribavirin, the therapeutic effect of ribavirin is also reduced. Thus, the therapeutic effect is lowered (Non Patent Literature 7). Moreover, recently it has been reported that in conventional standard treatments, the therapeutic effect is lower for an elderly patient (Non Patent Literature 3). The existence of a patient with chronic hepatitis C that is difficult to treat with currently-available drugs is a great medical issue to be addressed. In Japan, 70% or more of patients with chronic hepatitis C are patients with genotype 1b, and the percentage of elderly patients is also high.

The complete response rate (efficiency of obtaining the final therapeutic effect to completely remove a virus from blood) of the standard therapy (combination of IFN or PEG-IFN with ribavirin) for patients with chronic hepatitis C can be predicted more specifically using, as an index, a pattern of decreasing the blood HCV load or time when the blood HCV load is turned negative at a measureable sensitivity or lower after the treatment is started.

In this index, a case where negativity is achieved at 4 weeks from the beginning of the treatment is classified as "RVR (Rapid Virological Response)," and a case where the blood HCV load is decreased at 12 weeks from the beginning of the treatment to one hundredth (2 Log) or below of that before the administration is classified as "EVR (Early Virological Response)." The EVR is further classified into: a case where negativity is achieved at 12 weeks as "cEVR (Complete Early Virological Response) " and a case where the negativity is not achievedas "pEVR (Partial Early Virological Response)." Moreover, a case where negativity is achieved after 12 weeks and at 24 weeks from the beginning of the treatmentisclassified as"LVR (Late Virological Response)." A case where the blood HCV load is decreased at 12 weeks from the beginning of the treatment to one hundredth or below of that before the administration but the negativity is not achieved at 24 weeks is classified as "PR (Partial Responder). " A case where negativity is not achieved at 24 weeks from the beginning of the treatment is classified as "NR (Non Responder)." The Non Responder is further classified into: a case where the HCV load is not decreased at 24 weeks from the beginning of the treatment to one hundredth or below of that before the administration as "Null Responder" (Non Patent Literature 8).

The relationship between these classifications and the complete response rate is as follows. For example, if a treatment is performed for 48 weeks using a combination of PEG-IFN with ribavirin, the complete response rates for patients with genotype 1 and having a high viral load are: 90 to 100% in the RVR case, 71 to 75% in the cEVR case, 36 to 45% in the LVR case, and 0 to 2% in a case where negativity is achieved after 24 weeks (Non Patent Literatures 7, 9). To put it differently, a patient with genotype 1 and having a high viral load, particularly in the case where negativity is achieved after 12 weeks, is a refractory patient with a low complete response rate by the standard therapy.

Recently, researches have proceeded on drugs directly inhibiting HCV proliferation as therapeutic drugs for patients with chronic hepatitis C including refractory chronic hepatitis C cases. These drugs are additionally added to a combination of an IFN with ribavirin in the standard therapy and are characterized by being used in a combination in a three or multiple dosage form. The drugs are expected to quickly remove HCV from bodies of patients with chronic hepatitis C including refractory chronic hepatitis C cases. However, issues yet to be solved are pointed out on the drugs directly inhibiting HCV proliferation, such as that the drugs induce HCV to have the drug resistance, and that the treatment cannot be accomplished by inherent side effects of the drugs. At present, no drugs that can be used normally are available for refractory chronic hepatitis C cases (Non Patent Literature 10). Additionally, to expect high therapeutic effects, combination with ribavirin is necessary, and the problem with ribavirin side effects is inevitable. For this reason, highly safe and effective drugs are strongly demanded for patients with refractory chronic hepatitis C in the medical field.

Meanwhile, it is reported that 22β-methoxyolean-12-ene-3β,24(4β)-diol having an inhibitory effect on hepatocyte disorder has a synergistic anti-HCV activity in combination with an IFN. This compound is expected to form a drug excellent for preventing or treating viral diseases when administered in a combination in a two dosage form not including ribavirin (Patent Literature 1).

However, effectiveness and safety for patients with refractory chronic hepatitis C have not been reported yet so far.

### [Citation List]

### [Patent Literature]

[Patent Literature 1] International Publication No. W02008/004653

### [Non Patent Literature]

[NPL 1] "New England Journal of Medicine," (US), 2001, vol. 345, No. 1, pp. 41-52
[NPL 2] "Journal of Clinical Microbiology," (US), 2006, pp. 318-323
[NPL 3] "New problems in Hepatitis B and C treatments," Medical Journal sya. Co., Ltd., 2008, pp. 19-33
[NPL 4] "New treatment method for viral hepatitis, " Medical View Co., Ltd., 2008, vol. 25, no. 3, pp. 76-81
[NPL 5] "Rebetol Capsules 200 mg," revised in February, 2007 (7th) attached document, Schering-Plough K. K.
[NPL 6] "Copegus tablets 200 mg," revised in July, 2007 (4th) attached document, Chugai Pharmaceutical Co., Ltd.
[NPL 7] "Clinical pharmacology" in Kan-tan-sui, 2004, 49(6), pp. 1099-1121
[NPL 8] "Hepatology," (US), 2009, vol. 49, no. 4, pp. 1335-1374
[NPL 9] "Antiviral Therapy," (US), 2008, vol. 13, pp. 9-16
[NPL 10] "Nature Reviews," (US), 2007, vol. 6, pp. 991-1000

### [Summary of Invention]

### [Technical Problem]

The present invention has been made in view of such circumstances. An object of the present invention is to provide an agent effective for refractory patients with chronic hepatitis C and having few side effects. Another object of the present invention is to provide a method for treating refractory chronic hepatitis C using such an agent.

### [Solution to Problem]

The present inventors have earnestly studied in order to solve the above problems. As a result, it has been found out that by administering an effective amount of a combination of 22β-methoxyolean-12-ene-3β,24(4β)-diol with an IFN as active ingredients, even refractory chronic hepatitis C can be effectively treated. Particularly, this combination exhibited an excellent effect on a patient who relapsed after the blood HCV load was turned negative at 24 weeks from a standard combination therapy of an IFN with ribavirin. Moreover, the combination also exhibited an excellent effect on a refractory patient, in the standard combination therapy of an IFN with ribavirin, whose blood HCV load was not turned negative at 24 weeks but was turned negative after 24 weeks, and then who relapsed and had a high viral load of the blood HCV, or a refractory patient whose blood HCV load was not turned negative even after 24 weeks and was a high viral load. Further, the present inventors have found out that this combination, when administered, prevents decrease in blood hemoglobin, which otherwise causes anemia, in comparison with the standard therapy.

Specifically, the present inventors have found out that a medicine comprising a combination of 22β-methoxyolean-12-ene-3β,24(4β)-diol with an IFN as active ingredients is effective against refractory chronic hepatitis C and excellent in safety, and thus completed the present invention.

The present invention more specifically provides the following inventions.
(1) An agent for treating refractory chronic hepatitis C, the agent comprising a combination of 22β-methoxyolean-12-ene-3β,24(4β)-diol with an interferon as active ingredients.
(2) The agent according to (1), targeting a patient having a genotype 1b hepatitis C virus in blood thereof.
(3) The agent according to (2), targeting the patient having a high viral load of the hepatitis C virus in the blood.
(4) A combination **characterized in that**
   the combination comprises:
   the agent according to (1); and
   an agent comprising a combination of an interferon with ribavirin as active ingredients,
      (a) the agent comprising the combination of the interferon with the ribavirin as the active ingredients is administered to a patient with refractory chronic hepatitis C, and
      (b) when a load of a hepatitis C virus in blood of the patient is turned negative after 12 weeks from the administration but is turned to a high viral load after the negativity is achieved, the agent according to (1) is administered.
(5) The combination according to (4), wherein the agent according to (1) is administered when the load of the hepatitis C virus in the blood of the patient is turned negative at 12 weeks to 24 weeks but is turned to a high viral load after after the negativity is achieved.
(6) A combination **characterized in that** the combination comprises:
   the agent according to (1); and
   an agent comprising a combination of an interferon with ribavirin as active ingredients,
      (a) the agent comprising the combination of the interferon with the ribavirin as the active ingredients is administered to a patient with refractory chronic hepatitis C, and
      (b) when a load of a hepatitis C virus in blood of the patient is turned negative after 24 weeks from the administration but is turned to a high viral load after the negativity is achieved, or when the load is not turned negative even after 24 weeks from the administration, the agent according to (1) is administered.
(7) A combination **characterized in that** the combination comprises:
   the agent according to (1); and
   an agent comprising a combination of an interferon with ribavirin as active ingredients,
      (a) the agent comprising the combination of the interferon with the ribavirin as the active ingredients is administered to a patient with refractory chronic hepatitis C, and
      (b) then, when a load of a hepatitis C virus in blood of the patient is decreased and a hemoglobin value in the blood of the patient is low, the agent according to (1) is administered.
(8) A method for treating refractory chronic hepatitis C, the method comprising administering an effective amount of a combination of 22β-methoxyolean-12-ene-3β,24(4β)-diol with an interferon.
(9) The method according to (8), targeting a patient having a genotype 1b hepatitis C virus in blood thereof.
(10) The method according to (9), targeting the patient having a high viral load of the hepatitis C virus in the blood.
(11) A method for treating refractory chronic hepatitis C, the method characterized by comprising:
   (a) administering to a patient an agent comprising a combination of an interferon with ribavirin as active ingredients; and
   (b) when a load of a hepatitis C virus in blood of the patient is turned negative after 12 weeks from the administration but is turned to a high viral load after the negativity is achieved, administering the agent according to (1).
(12) The method according to (11), wherein the agent according to (1) is administered when the load of the hepatitis C virus in the blood of the patient is turned negative at 12 weeks to 24 weeks but is turned to a high viral load after the negativity is achieved.
(13) A method for treating refractory chronic hepatitis C, the method characterized by comprising:
   (a) administering to a patient an agent comprising a combination of an interferon with ribavirin as active ingredients; and
   (b) when a load of a hepatitis C virus in blood of the patient is turned negative after 24 weeks from the administration but is turned to a high viral load after the negativity is achieved, or when the load is not turned negative even after 24 weeks from the administration, administering the agent according to (1).
(14) A method for treating refractory chronic hepatitis C, the method characterized by comprising:
   (a) administering to a patient an agent comprising a combination of an interferon with ribavirin as active ingredients; and
   (b) then, when a load of a hepatitis C virus in blood of the patient is decreased and a hemoglobin value in the blood of the patient is low, administering the agent according to (1).

### [Advantageous Effects of Invention]

The present invention provides: an agent for treating refractory chronic hepatitis C, the agent comprising 22β-methoxyolean-12-ene-3β,24(4β)-diol and an IFN as active ingredients; and a method for treating refractory chronic hepatitis C using the agent. The agent of the present invention comprising the 22β-methoxyolean-12-ene-3β,24(4β)-diol and the IFN as the active ingredients exhibits excellent effects against refractory chronic hepatitis C. Moreover, by the treatment with the agent of the present invention, a problem with the side effects such as anemia in the standard therapy is significantly suppressed. The present invention makes it possible to provide effective and safe medical treatment for patients with refractory chronic hepatitis C.

### [Brief Description of Drawings]

[Fig. 1] Fig. 1 is a graph showing changes inbloodhemoglobin values until 12 weeks in a standard combination therapy of ribavirin with PEG-IFN and in a therapy of the present invention using a combination of 22β-methoxyolean-12-ene-3β,24(4β)-diol with PEG-IFN.
[Fig. 2] Fig. 2 is a graph showing changes in blood hemoglobin values until 24 weeks in the standard combination therapy of the ribavirin with the PEG-IFN and in the therapy of the present invention using the combination of the 22β-methoxyolean-12-ene-3β,24(4β)-diol with the PEG-IFN.

### [Description of Embodiments]

The present invention provides: an agent for treating refractory chronic hepatitis C, the agent comprising a combination of 22β-methoxyolean-12-ene-3β,24(4β)-diol with an IFN as active ingredients; and a method for treating refractory chronic hepatitis C, the method comprising administering an effective amount of the combination.

In the present invention, "chronic hepatitis C" means an inflammatory disease in the liver caused by persistent infection of a hepatitis C virus. In such Hepatitis C, the infection persists for 6 months or longer. In addition, "refractory chronic hepatitis C" means a type of chronic hepatitis C that makes complete recovery difficult by a standard combination therapy of an IFN with ribavirin. Typically, the hepatitis C virus in blood is genotype 1b, and more typically a patient has a high viral load. Herein, the "high viral load" means a case where the amount of virus in blood meets any baseline of 1 Meq/mL or higher, 5.0 Log IU/mL (100 KIU/mL) or higher, and 300 fmol/L or higher. A patient having a high viral load of the hepatitis C virus in the blood has a nature of a low complete response rate if the blood viral load is turned negative after 12 weeks from the beginning of the treatment by the standard therapy. Additionally, the following patient has a nature of a particularly low complete response rate: a patient whose blood HCV load is not turned negative at 24 weeks from the beginning of the treatment by the standard therapy but is turned negative after 24 weeks, and then who relapses and has a high viral load of the blood HCV; or a patient whose blood HCV load is not turned negative even after 24 weeks and is a high viral load.

Meanwhile, when a blood hemoglobin value is low, a patient has a nature that the standard treatment including ribavirin cannot be started or accomplished, and the blood viral load is not turned negative. Herein, the phrase "when the hemoglobin value is low" generally means a case where the hemoglobin value is below 10 g/dL. It should be noted, however, that in a case where a patient has a heart disease now or in the past, the phrase "when the hemoglobin value is low" also includes meanings of: a prolonged state, even if the hemoglobin value is 10 g/dL or above, where the hemoglobin value drops down 2 g/dL or more after the standard treatment including ribavirin is started (for example, such a state is prolonged for 4 weeks or longer) ; and a prolonged state where the hemoglobin value is below 12 g/dL after the administration dose of the ribavirin in the standard therapy is reduced because the hemoglobin value has been below 10 g/dL (for example, such a state is prolonged for 4 weeks or longer).

The "22β-methoxyolean-12-ene-3β,24(4β)-diol" used as the active ingredient in the agent of the present invention is a known compound, and can be obtained, for example, by the method described in Example 22 (compound 27)of International Publication WO97/03088. In general, the 22β-methoxyolean-12-ene-3β,24(4β)-diol may be orally administered as a conventional pharmaceutical formulation, for example, capsules, microcapsules, tablets, granules, fine granules, powders, and the like. Further, the compound may be parenterally administered (for example, intranevenous, intramuscular, subcutaneous administration, intraperitoneal administration, rectal administration, and percutaneous administration) as a conventional pharmaceutical formulation by, for example, intranevenous injection, intramuscular injection, or other means. These formulations can be prepared by a conventional method using generally-used pharmaceutically acceptable additives such as an excipient, a filler, a binder, a wetting agent, a disintegrating agent, a surfactant, a lubricant, a dispersant, a buffer, a preservative, a solubilizer, an antiseptic, a flavor, a soothing agent, and a stabilizer. Specific examples of the above additives include lactose, fructose, glucose, starch, gelatin, magnesium carbonate, synthetic magnesium silicate, talc, magnesium stearate, methyl cellulose, carboxymethyl cellulose or a salt thereof, gum arabic, polyethylene glycol, syrup, vaseline, glycerin, ethanol, propylene glycol, citric acid, sodium chloride, sodium sulfite, sodium phosphate, and the like.

In the agent of the present invention, the dosage form, the administration method, the administration dose, the administration period, the administration interval, the administration route, and the like of the 22β-methoxyolean-12-ene-3β,24(4β)-diol may be appropriately set in accordance with, for example, the weight, the age, the blood viral load, the severity of symptoms, and the like of a patient. These are not particularly limited, so long as the antiviral effect is produced when combined with the IFN. For example, a daily dose of 1 to 1000 mg is orally or parenterally administered as a single dose or plural divided doses. Preferably, a daily dose of 25 to 800 mg is divided into two doses, which are orally or parenterally administered.

Examples of the "interferon" used as the active ingredient in the agent of the present invention include, without limitation to, IFN derivatives used in the treatment, such as natural IFN-α (Sumiferon: manufactured by Dainippon Sumitomo Pharma Co. , Ltd. , and the like), IFN-α-2a, IFN-α-2b (Intron A: manufactured by Schering-Plough K. K.), PEG-natural IFN-α, PEG-IFN-α-2a (Pegasys: manufactured by Roche Holding AG and Chugai Pharmaceutical Co., Ltd.), PEG-IFN-α-2b (PEG-Intron A: manufactured by Schering-Plough K. K.), natural IFN-β (IFNβ Mochida: manufactured by Mochida Pharmaceutical Co., Ltd., and Feron: manufactured by Toray Industries, Inc.), PEG-IFN-β, natural IFN-γ, consensus IFN (Advaferon: manufactured by Astellas Pharma Inc., and the like), PEG-consensus IFN, and long-lasting IFN. Preferable are IFN-α-2b and IFN-α-2a, and more preferable are those PEGylated.

In the agent of the present invention, the dosage form, the administration method, the administration dose, the administration period, the administration interval, the administration route, and the like of the IFN may be appropriately set in accordance with, for example, the weight, the age, the blood viral load, the severity of symptoms, and the like of a patient. These are not particularly limited, so long as the antiviral effect is produced when combined with the 22β-methoxyolean-12-ene-3β,24(4β)-diol. In the treatment guideline in Japan, for a case of genotype 1b with a high viral load, basically, PEG-IFN and ribavirin are administered in combination for 48 weeks. For some cases, it is allowed to select extension of the administration up to 72 weeks so as to increase the therapeutic effect. The administration period of the IFN in the agent of the present invention is not necessarily limited to the period according to this guideline. The administration period can be appropriately modified in accordance with the type or the dosage form of the IFN to be administered. Moreover, although normally subcutaneously, intravenously or intramuscularly administered, the IFN may be administered by another parenteral method (for example, by a nasal spray, through skin, in a suppository, or the like) or an oral method using an oral IFN, if medically effective. For example, normally, a daily dose of 6,000,000 to 10,000,000 IU of the IFN is administered continuously for 2 weeks to 8 weeks followed by intermittent administration for 22 weeks to 46 weeks. Alternatively, the IFN is administered continuously for 48 weeks or longer at 180 µg/person for PEG-IFN-α-2a or 1.5 µg/kg for PEG-IFN-α-2b by subcutaneous administration per week.

In the agent of the present invention, the combination of the 22β-methoxyolean-12-ene-3β,24(4β)-diol with the IFN is not particularly limited, so long as the combination is is effective in the treatment against refractory chronic hepatitis C. For example, as the IFN, 180 µg of PEG-IFN-α-2a or 1.5 µg/kg of PEGylated IFN-α-2b is administered per week, while a daily dose of the 22β-methoxyolean-12-ene-3β,24(4β)-diol is determined appropriately within a range of 1 mg to 1000 mg, and 25 mg to 800 mg is administered a day. Appropriate administration dose and administration interval of each of the 22β-methoxyolean-12-ene-3β,24(4β)-diol and the IFN can be determined in accordance with a controlled clinical trial.

When the agent of the present invention is administered, the 22β-methoxyolean-12-ene-3β,24(4β) -diol and the IFN are administered in combination. The term "combination" in the present invention includes administration of each of single preparations simultaneously or with a time interval. Moreover, the number of administrations of each component may be the same as or different from that of the other. Thus, the agent of the present invention may be an agent in a single dosage form comprising the two active ingredients in one composition, or an agent in two dosage forms comprising the two active ingredients in separate compositions.

The agent of the present invention may be administered to a patient after application of a standard therapy (combination of an IFN with ribavirin). As the administration mode of the agent of the present invention, first, an agent comprising a combination of an IFN with ribavirin as active ingredients is administered to a patient; then, when negativity is achieved after 12 weeks from the administration by the effect of the combination of the IFN with the ribavirin but a high viral load is observed after the negativity is achieved, the agent of the present invention is administered. Moreover, after the agent comprising the combination of the IFN with the ribavirin as the active ingredients is administered to a patient, and when negativity is achieved after 24 weeks after the administration by the effect of the combination of the IFN with the ribavirin but a high viral load is observedafter the negativity is achieved, or when negativity is not achieved even after 24 weeks from the administration, the agent of the present invention is administered. Further, when the load of hepatitis C virus in blood of a patient is decreased by the effect of the combination of the IFN with the ribavirin but it is difficult to continue the administration at a standard administration dose because the blood hemoglobin value of the patient is low (i.e., because of the side effects such as anemia), the agent of the present invention is administered. Herein, the phrase "when the hemoglobin value is low" generally means a case where the hemoglobin value is below 10 g/dL. It should be noted, however, that in a case where a patient has a heart disease now or in the past, the phrase "when the hemoglobin value is low" also includes meanings of: a prolonged state, even if the hemoglobin value is 10 g/dL or above, where the hemoglobin value drops down 2 g/dL or more after the ribavirin administration is started (for example, such a state is prolonged for 4 weeks or longer) ; and a prolonged state where the hemoglobin value is below 12 g/dL after the administration dose of the ribavirin in the standard therapy is reduced because the hemoglobin value has been below 10 g/dL (for example, such a state is prolonged for 4 weeks or longer). The patient who is a target of the administration of the agent of the present invention is preferably: a patient whose load of hepatitis C virus in the blood is turned negative at 12 weeks to 24 weeks from the administration of the agent comprising the combination of the IFN with the ribavirin as the active ingredients but is turned to a high viral load after the negativity is achieved; a patient whose load of hepatitis C virus is turned negative after 24 weeks from the administration of the agent comprising the combination of the IFN with the ribavirin as the active ingredients but is turned to a high viral load after the negativity is achieved; or a patient whose load of hepatitis C virus is not turned negative even after 24 weeks from the administration of the agent comprising the combination of the IFN with the ribavirin as the active ingredients. In this manner, the agent of the present invention is also applicable in combination with the standard therapy to a patient.

### [Examples]

Hereinafter, the present invention will be specifically described by way of Examples, but the scope of the present invention is not limited thereto.

### [Example 1] Examination of antiviral effect of 22β-methoxyolean-12-ene-3β,24(4β)-diol and PEG-IFN in refractory chronic hepatitis C case (LVR case)

Subjects were 21 patients (9 men, 12 women) infected with genotype 1b HCV and having chronic hepatitis C. The patients had been subjected to a combination therapy of PEG-IFN with ribavirin for 48 weeks that is a standard treatment against genotype 1b chronic hepatitis C with a high viral load. The blood HCV load was not turned negative at 12 weeks but the blood HCV load was turned negative at 24 weeks (LVR case). Thereafter, the patients relapsed and the blood HCV load was turned to a high viral load. To these subject patients in a refractory chronic hepatitis C case, a fine granule containing 25 mg or 400 mg of 22β-methoxyolean-12-ene-3β,24(4β)-diol was orally administered twice a day and 180 µg of PEG-IFN-α-2a (Pegasys : manufactured by Roche Holding AG and Chugai Pharmaceutical Co., Ltd.) was subcutaneously administered per week. These administrations were continued for 12 weeks (hereinafter, the patients were referred to as a "50 mg/day administration group" or an "800 mg/day administration group"). The transitions of the blood HCV load and safety (clinical test value abnormality: blood hemoglobin value) before the administrations and up to 12 weeks were examined. The blood HCV load was measured using the TaqMan^{(R)} PCR method, and the baseline of negativity was below 1.2 Log IU/mL.

Table 1 shows the backgrounds of the patients in the 50 mg/day administration group and the 800 mg/day administration group. The backgrounds of all the patients in the two administration groups are as follows. As for the sex, there were 9 men (42.9%) and 12 women (57.1%). As for the age, the average age was 59.1, the youngest was 35 years old, and the oldest was 67 years old. With the baseline of 60 years old, there were 9 people who were under 60 years old (42.9%) and 12 people who were 60 years older above (57.1%). The average blood HCV load of the patients before the administrations was 6.37 Log IU/mL with the minimum load of 5.00 Log IU/mL and the maximum load of 7.20 Log IU/mL. These exceeded the baseline, 5.00 Log IU/mL, of the high viral load, and all the patients were infected with genotype 1b.

**[Table 1]**

| Patient backgrounds | | | | |
|---|---|---|---|---|
| Items | | 50 mg/day group (N=11) | 800 mg/day group (N=10) | Total (N=21) |
| Sex | Women | 6 (54.5%) | 6 (60.0%) | 12 (57.1%) |
| | Men | 5 (45.5%) | 4 (40.0%) | 9 (42.9%) |
| Age (year) | Average | 61.18 | 56.30 | 59.10 |
| | Youngest | 52.00 | 35.00 | 35.00 |
| | Oldest | 67.00 | 67.00 | 67.00 |
| Age (frequency) | ∼< 60 years old | 3 (27.3%) | 6 (60.0%) | 9 (42.9%) |
| | 60 years old- | 8 (72.7%) | 4 (40.0%) | 12 (57.1%) |
| Weight (kg) | Average | 61.79 | 62.14 | 61.96 |
| | Heaviest | 50.60 | 51.10 | 50.10 |
| | Lightest | 73.60 | 76.20 | 76.20 |
| HCV>RNA before administrations Log (IU/mL)) | Average | 6.22 | 6.53 | 6.37 |
| | Minimum | 5.00 | 5.60 | 5.00 |
| | Maximum | 7.00 | 7.20 | 7.20 |
| HCV-RNA before administrations (frequency) | ∼< 5.7 Log IU/mL | 2 (18.2%) | 1 (10.1%) | 3 (14.3%) |
| | 5.7 Log IU/mL ∼ | 9 (31.8%) | 9 (90.0%) | 13 (85.7%) |
| | ∼< 6.7 Log IU/mL | 8 (72.7%) | 5 (50.0%) | 13 (61.9%) |
| | 6.7 Log IU/mL ∼ | 3 (27.3%) | 5 (50.0%) | 8 (38.1%) |

Table 2 shows the ratio of negativity achieved in the blood HCV load at 12 weeks. In the standard combination therapy of PEG-IFN with ribavirin performed as the pretreatment, the ratio of negativity achieved in the blood HCV load at 12 weeks was 0%. In contrast, in all the administration groups of 22β-methoxyolean-12-ene-3β,24(4β)-diol with PEG-IFN-α-2a, 57.1% was turned negative.

**[Table 2]**

| Ratio of *negativity achieved in blood HCV after 12 weeks from administrations | | | |
|---|---|---|---|
| Items | | Ratio of negativity achieved | Ratio of negativity achieved in pretreatment of PEG-IFN + ribavirin |
| Administration groups | 50 mg/day | 63.6% (7/11) | 0% (0/11) |
| | 800 mg/day | 50.0% (5/10) | 0% (0/10) |
| | Total | 57.1% (12/21) | 0% (0/21) |

Table 3 shows the result categorized by the sex. In both men and women, the ratio of negativity achieved in the blood HCV load at 12 weeks significantly exceeded the ratio of negativity achieved in the pretreatment of 0%.

**[Table 3]**

| Ratio of negativity achieved in blood HCV after 12 weeks from administrations (sex) | | |
|---|---|---|
| Items | | Ratio of negativity achieved |
| Men | 60 mg/day | 60.0% (3/5) |
| | 600 mg/day | 60.0% (2/4) |
| | Total | 88.7% (4/6) |
| Women | 50 mg/day | 88.7% (4/6) |
| | 800 mg/day | 80.0% (3/6) |
| | Total | 68.3% (7/12) |

Table 4 shows the result categorized by the age. In any of those who were under 60 years old and who were 60 years old or above, the ratio of negativity achieved in the blood HCV load at 12 weeks significantly exceeded the ratio of negativity achieved in the pretreatment of 0%.

**[Table 4]**

| Ratio of negativity achieved an blood HCV after 12 weeks from administrations (age) | | |
|---|---|---|
| Items | | Ratio of negativity achieved |
| ∼<60 years old | 50 mg/day | 33.3% (1/3) |
| | 800 mg/day | 66.7% (4/6) |
| | Total | 55.6% (8/9) |
| 60 years old∼ | 50 mg/day | 75.0% (6/8) |
| | 800 mg/day | 25.0% (1/4) |
| | Total | 88.3% (7/12) |

Note that, in the above-described case where the fine granule containing 25 mg or 400 mg of 22β-methoxyolean-12-ene-3β,24(4β)-diol was orally administered twice a day and 180 µg of PEG-IFN-α-2a (Pegasys : manufactured by Roche Holding AG and Chugai Pharmaceutical Co., Ltd.) was subcutaneously administered per week, the blood HCV load was turned negative by 12 weeks. In a subsequent case where the administrations were continued up to 48 weeks, in two out of three patients, the blood HCV load was continued to be negative for 48 weeks of the administration period. In this case, no relapse occurred even after the non-treatment period of subsequent 24 weeks, and complete recovery was achieved.

### [Example 2] Examination of influence of 22β-methoxyolean-12-ene-3β,24(4β)-diol and PEG-IFN on blood hemoglobin value in refractory chronic hepatitis C case (LVR case)

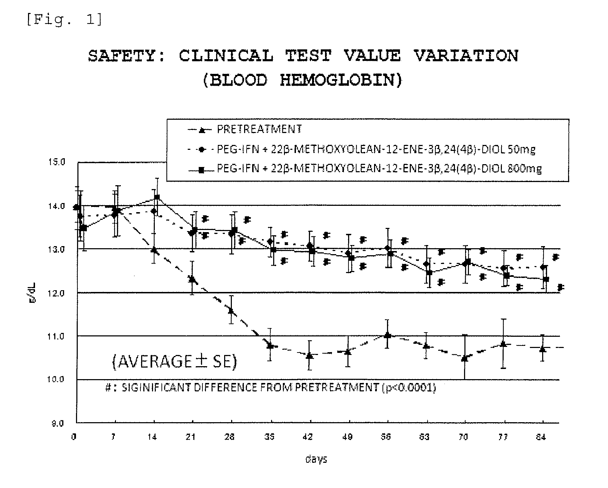

Fig. 1 shows changes in blood hemoglobin values of the patients. In the standard combination therapy of PEG-IFN with ribavirin performed as the pretreatment, it was observed that the blood hemoglobin value was apparently decreased to 11.0 g/dL or below on average by 5 weeks (35 days) from the administration. In contrast, in the administrations of 22β-methoxyolean-12-ene-3β,24(4β)-diol and PEG-IFN-α-2a, the blood hemoglobin value did not reach 12.0 g/dL or below on average at any dose, and the decrease in the hemoglobin value was apparently low.

As described above, when ribavirin is used in the treatment, whether the blood hemoglobin value is 12 g/dL or above has to be checked before the treatment is started. If the blood hemoglobin value is therebelow, the amount of ribavirin administered is reduced or the administration is terminated. Nevertheless, in the method of the present invention in which 22β-methoxyolean-12-ene-3β,24(4β) -diol is used in combination with an IFN, such a decrease in hemoglobin was not observed. Thus, there were no patients who discontinued or abandoned the treatment.

### [Example 3] Examination of antiviral effect of 22β-methoxyolean-12-ene-3β,24(4β)-diol and PEG-IFN in refractory chronic hepatitis C case (NR case)

Subjects were the following patients (12 in total; 7 men, 5 women) who had been infected with genotype 1b HCV and had chronic hepatitis C and who were particularly considered to be highly refractory.
(1) Patients who were subjected to a combination therapy of PEG-IFN with ribavirin for 48 weeks that is a standard treatment against genotype 1b chronic hepatitis C with a high viral load, whose blood HCV load was not turned negative at 24 weeks but was turned negative after 24 weeks, and then who relapsed and had a high viral load of the blood HCV (NR case).
(2) Patients whose blood HCV load was decreased at 12 weeks from the beginning of the treatment to one hundredth or below of that before the administration but was not turned negative even after 24 weeks (PR case), and who had a high viral load of the blood HCV (NR case).

To these subject patients (NR case), a fine granule containing 25 mg or 400 mg of 22β-methoxyolean-12-ene-3β,24(4β)-diol was orally administered twice a day and 180 µg of PEG-IFN-α-2a (Pegasys : Roche Holding AG and Chugai Pharmaceutical Co., Ltd.) was subcutaneously administered per week. These administrations were continued for 24 weeks (hereinafter, the patients were referred to as a "50 mg/day administration group" or an "800 mg/day administration group"). The transitions of the blood HCV load and safety (clinical test value abnormality: blood hemoglobin value) before the administrations and up to 24 weeks were examined. The blood HCV load was measured using the TaqMan^{(R)} PCR method, and the baseline of negativity was below 1.2 Log IU/mL.

Table 5 shows the backgrounds of the patients in the 50 mg/day administration group and the 800 mg/day administration group. The backgrounds of all the patients in the two administration groups are as follows. As for the sex, there were 7 men (58.3%) and 5 women (47.1%). As for the age, the average age was 58.3, the youngest was 43 years old, and the oldest was 69 years old. With the baseline of 60 years old, there were 6 people who were under 60 years old (50.0%) and 6 people who were 60 years old or above (50.0%). The average blood HCV load of the patients before the administrations was 6.66 Log IU/mL with the minimum load of 5.30 Log IU/mL and the maximum load of 7.40 Log IU/mL. These exceeded the baseline, 5.00 Log IU/mL, of the high viral load, and all the patients were infected with genotype 1b.

**[Table 5]**

| Patient backgrounds | | | | |
|---|---|---|---|---|
| Items | | 50 mg/day group (N=6) | 800 mg/day group (N=6) | Total (N=12) |
| Sex | Women | 2 (33.3%) | 3 (50.0%) | 5 (41.7%) |
| | Men | 4 (66.7%) | 3 (50.0%) | 7 (58.3%) |
| Age (year) | Average | 56.33 | 59.50 | 58.17 |
| | Youngest | 43.00 | 44.00 | 43.00 |
| | Oldest | 67.00 | 69.00 | 69.00 |
| Age (frequency) | ∼< 60 years old | 4 (66.7%) | 2 (33.3%) | 6 (50.0%) |
| | 60 years old∼ | 2 (33.3) | 4 (66.7%) | 6 (50.0%) |
| Weight | Average | 65.64 | 59.23 | 62.44 |
| | Heaviest | 47.30 | 47.90 | 47.90 |
| | Lightest | 84.90 | 76.50 | 84.90 |
| HCV-RNA before administrations (Log (IU/mL)) | Average | 6.52 | 6.80 | 6.66 |
| | Minimum | 5.30 | 5.60 | 5.30 |
| | Maximun | 7.20 | 7.40 | 7.40 |
| HCV-RNA before administrations (frequency) | ∼< 5.7 Log IU/mL | 1 (16.7%) | 1 (16.7%) | 2 (16.7&) |
| | 5.7 Log IU/mL | 5 (83.3%) | 5 (83.3%) | 10 (83.3%) |
| | < 6.7 Log IU/mL | 3 (50.0%) | 1 (16.7%) | 4 (33.3%) |
| | 6.7Log IU/mL | 3 (50.0%) | 5 (33.3%) | 8 (66.7%) |

Table 6 shows the ratio of negativity achieved in the blood HCV load at 24 weeks. In the standard combination therapy of PEG-IFN with ribavirin performed as the pretreatment, the ratio of negativity achieved in the blood HCV load at 24 weeks was 0%. In contrast, in all the administration groups of 22β-methoxyolean-12-ene-3β,24(4β)-diol with PEG-IFN-α-2a, 50.0% was turned negative.

**[Table 6]**

| Ratio of negativity achieved in blood HCV after 24 weeks from administrations | | | |
|---|---|---|---|
| Items | | Ratio of negativity achieved | Ratio of negativity achieved in pretreatment of PEG-IFN + ribavirin |
| Administration groups | 50 mg/day | 50.0% (3/6) | 0% (0/6) |
| | 800 mg/day | 50.0% (3/6) | 0% (0/6) |
| | Total | 60.0% (6/12) | 0% (0/12) |

Table 7 shows the result categorized by the sex. In both men and women, the ratio of negativity achieved in the blood HCV load at 24 weeks significantly exceeded the ratio of negativity achieved in the pretreatment of 0%.

**[Table 7]**

| Ratio of negativity achieved in blood HCV after 24 weeks from administrations (sex) | | |
|---|---|---|
| Items | | Ratio of negativity achieved |
| Men | 50 mg/day | 50.0% (2/4) |
| | 800 mg/day | 33.3% (1/3) |
| | Total | 42.9% (3/7) |
| Women | 50 mg/day | 60.0% (1/2) |
| | 800 mg/day | 66.7% (2/3) |
| | Total | 60.0% (3/6) |

Table 8 shows the result categorized by the age. In any of those who were under 60 years old and who were 60 years old or above, the ratio of negativity achieved in the blood HCV load at 24 weeks significantly exceeded the ratio of negativity achieved in the pretreatment of 0%.

**[Table 8]**

| Ratio of negativity achieved in blood HCV after 24 weeks from administrations (age) | | |
|---|---|---|
| Items | | Ratio of negativity achieved |
| ∼< 60 years old | 50 mg/day | 50.0% (2/4) |
| | 800 mg/day | 50.1% (1/2) |
| | Total | 50.0% (3/6) |
| 60 years old∼ | 50 mg/day | 50.0% (1/2) |
| | 800 mg/day | 50.0% (2/4) |
| | Total | 50.0% (3/6) |

### [Example 4] Examination of influence of 22β-methoxyolean-12-ene-3β,24(4β) -diol andPEG-IFNonblood hemoglobin value in refractory chronic hepatitis C case (NR case)

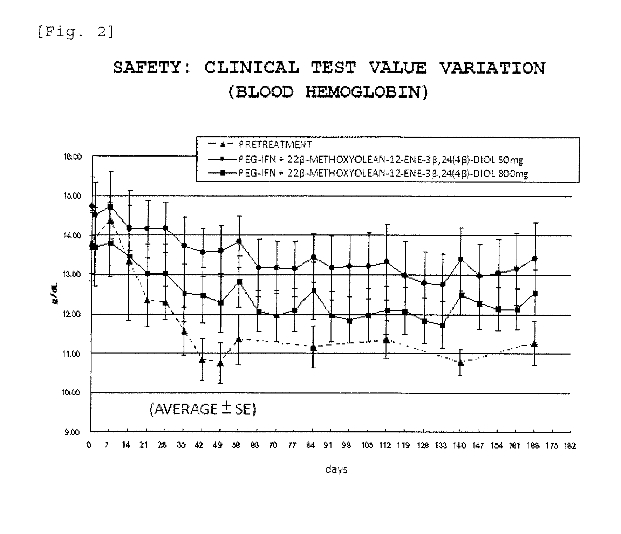

Fig. 2 shows changes in blood hemoglobin values of the patients. In the standard combination therapy of PEG-IFN with ribavirin as the pretreatment, it was observed that the blood hemoglobin value was apparently decreased to approximately 11.0 g/dL on average by 24 weeks (168 days) from the administration. On the other hand, in the administrations of 22β-methoxyolean-12-ene-3β,24(4β) -diol and PEG-IFN-α-2a, the blood hemoglobin value did not reach 12.0 g/dL or below on average at the dose of 50 mg. In addition, although the blood hemoglobin value reached 12.0 g/dL or below on average at the dose of 800 mg, these patients had the blood hemoglobin value of 14.0 g/dL or below on average before the treatment was started, and the extent of the decrease was small; therefore, the decrease in the hemoglobin value was small.

As described above, when ribavirin is used in the treatment, whether the blood hemoglobin value is 12 g/dL or above has to be checked before the treatment is started. If the blood hemoglobin value is therebelow, the amount of ribavirin administered is reduced or the administration is terminated. Nevertheless, in the method of the present invention in which 22β-methoxyolean-12-ene-3β,24(4β)-diol is used in combination with an IFN, although a decrease in hemoglobin was observed at the dose of 800 mg, the decrease was small. Thus, there were no patients who discontinued or abandoned the treatment.

### [Industrial Applicability]

An agent of the present invention can exhibit high therapeutic effects for: a patient with chronic hepatitis C whose blood viral load (HCV RNA) shows a high viral load; particularly a patient with chronic hepatitis C having genotype 1b virus (HCV RNA) in the blood; or a patient with chronic hepatitis C who relapses after application of a standard combination therapy of an IFN with ribavirin. Moreover, in treatment with the agent of the present invention, a problem with side effects such as anemia in the standard therapy is significantly reduced. The agent of the present invention, because of high effectiveness and safety, is also suitable for a combination with an agent directly inhibiting HCV proliferation or other agents, and can also be expected to be applied to an emerging case of HCV subtypes which are extremely difficult to treat by conventional therapies.

## Claims

1. An agent for treating refractory chronic hepatitis C, the agent comprising a combination of 22β-methoxyolean-12-ene-3β,24(4β)-diol with an interferon as active ingredients.

2. The agent according to claim 1, targeting a patient having a genotype 1b hepatitis C virus in blood thereof.

3. The agent according to claim 2, targeting the patient having a high viral load of the hepatitis C virus in the blood.

4. A combination **characterized in that**
the combination comprises:
the agent according to claim 1; and
an agent comprising a combination of an interferon with ribavirin as active ingredients,
(a) the agent comprising the combination of the interferon with the ribavirin as the active ingredients is administered to a patient with refractory chronic hepatitis C, and
(b) when a load of a hepatitis C virus in blood of the patient is turned negative after 12 weeks from the administration but is turned to a high viral load after the negativity is achieved, the agent according to claim 1 is administered.

5. The combination according to claim 4, wherein the agent according to claim 1 is administered when the load of the hepatitis C virus in the blood of the patient is turned negative at 12 weeks to 24 weeks but is turned to a high viral load after the negativity is achieved.

6. A combination **characterized in that**
the combination comprises:
the agent according to claim 1; and
an agent comprising a combination of an interferon with ribavirin as active ingredients,
(a) the agent comprising the combination of the interferon with the ribavirin as the active ingredients is administered to a patient with refractory chronic hepatitis C, and
(b) when a load of a hepatitis C virus in blood of the patient is turned negative after 24 weeks from the administration but is turned to a high viral load after the negativity is achieved, or when the load is not turned negative even after 24 weeks from the administration, the agent according to claim 1 is administered.

7. A combination **characterized in that**
the combination comprises:
the agent according to claim 1; and
an agent comprising a combination of an interferon with ribavirin as active ingredients,
(a) the agent comprising the combination of the interferon with the ribavirin as the active ingredients is administered to a patient with refractory chronic hepatitis C, and
(b) then, when a load of a hepatitis C virus in blood of the patient is decreased and a hemoglobin value in the blood of the patient is low, the agent according to claim 1 is administered.

8. A method for treating refractory chronic hepatitis C, the method comprising administering an effective amount of a combination of 22β-methoxyolean-12-ene-3β,24(4β)-diol with an interferon.

9. The method according to claim 8, targeting a patient having a genotype 1b hepatitis C virus in blood thereof.

10. The method according to claim 9, targeting the patient having a high viral load of the hepatitis C virus in the blood.

11. A method for treating refractory chronic hepatitis C, the method **characterized by** comprising:
(a) administering to a patient an agent comprising a combination of an interferon with ribavirin as active ingredients; and
(b) when a load of a hepatitis C virus in blood of the patient is turned negative after 12 weeks from the administration but is turned to a high viral load after the negativity is achieved, administering the agent according to claim 1.

12. The method according to claim 11, wherein the agent according to claim 1 is administered when the load of the hepatitis C virus in the blood of the patient is turnednegative at 12 weeks to 24 weeks but is turned to a high viral load after the negativity is achieved.

13. A method for treating refractory chronic hepatitis C, the method **characterized by** comprising:
(a) administering to a patient an agent comprising a combination of an interferon with ribavirin as active ingredients; and
(b) when a load of a hepatitis C virus in blood of the patient is turned negative after 24 weeks from the administration but is turned to a high viral load after the negativity is achieved, or when the load is not turned negative even after 24 weeks from the administration, administering the agent according to claim 1.

14. A method for treating refractory chronic hepatitis C, the method **characterized by** comprising:
(a) administering to a patient an agent comprising a combination of an interferon with ribavirin as active ingredients; and
(b) then, when a load of a hepatitis C virus in blood of the patient is decreased and a hemoglobin value in the blood of the patient is low, administering the agent according to claim 1.
